(19) Europäisches Patentamt
European Patent Office
Office européen des brevets

(11) Veröffentlichungsnummer: **0 153 689**
**A2**

(12) # EUROPÄISCHE PATENTANMELDUNG

(21) Anmeldenummer: **85101803.6**

(22) Anmeldetag: **20.02.85**

(51) Int. Cl.⁴: **C 07 C 69/738**
C 07 C 69/734, C 07 F 7/00
C 07 D 307/93
//C07C177/00

(30) Priorität: **02.03.84 US 585751**

(43) Veröffentlichungstag der Anmeldung:
**04.09.85 Patentblatt 85/36**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Coffen, David Llewellyn**
**270 Ridgewood Avenue**
**Glen Ridge, N.J. 07028(US)**

(72) Erfinder: **Manchand, Percy S.**
**15 Prescott Avenue**
**Montclair, N.J. 07042(US)**

(72) Erfinder: **Truesdale, Larry K.**
**27 Gibbs Drive**
**Wayne, N.J. 07470(US)**

(74) Vertreter: **Grossner, Lutz, Dr. et al,**
**Grenzacher Strasse 124 Postfach 3255**
**CH-4002 Basel(CH)**

(54) **Prostaglandin-Zwischenprodukte.**

(57) Neue Prostaglandin-Zwischenprodukte der Formel

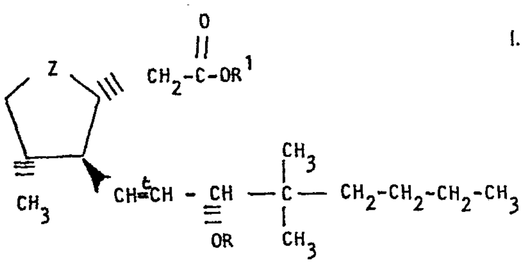

I.

worin Z >C=O oder

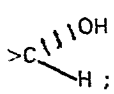

$R^1$ zusammen mit dem Sauerstoffatom, an das es gebunden ist, eine hydrolysierbare Estergruppe, R zusammen mit dem Sauerstoffatom, an das es gebunden ist, eine hydrolysierbare Aethergruppe darstellt und t trans-Konfiguration bezeichnet,

Diastereomere davon, in denen die ring-ständigen Substituenten die entgegengesetzte Stereokonfiguration zu der oben angegebenen haben, und Gemische davon, erhält man ausgehend von neuen Zirkon-Verbindungen und Cyclopentenylderivaten. Die Verbindungen der Formel I sind Zwischenprodukte für anti-sekretorisch wirksame Prostaglandine.

EP 0 153 689 A2

F. HOFFMANN-LA ROCHE & CO. Aktiengesellschaft, Basel/Schweiz

0153689

RAN 4303/13

# Prostaglandin-Zwischenprodukte

Die vorliegende Erfindung betrifft Prostaglandin-Zwischenprodukte und ein Verfahren zu deren Herstellung. Die Erfindung betrifft insbesondere Verbindungen der Formel

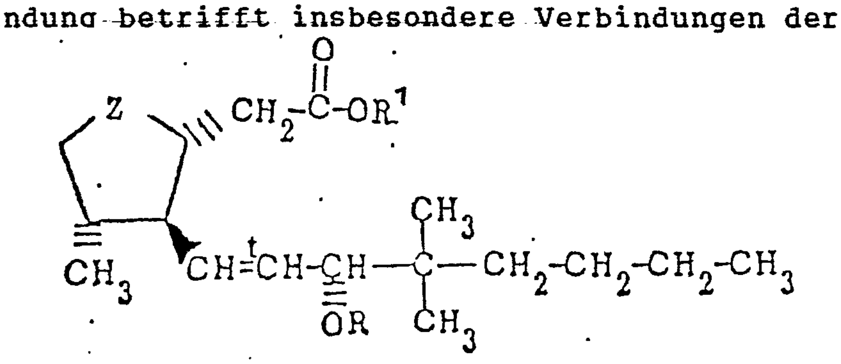

I.

worin Z >C=O oder 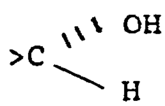 ; $R^1$ zusammen mit dem Sauerstoffatom, an das es gebunden ist, eine hydrolysierbare Estergruppe, R zusammen mit dem Sauerstoffatom, an das es gebunden ist, eine hydrolysierbare Aethergruppe darstellt und t trans-Konfiguration bezeichnet, Diastereomere davon, in denen die ring-ständigen Substituenten die entgegengesetzte Stereokonfiguration zu der oben angegebenen haben, und Gemische davon.

Grn/17.1.85

Der hier verwendete Ausdruck "nieder Alkyl" bezieht sich auf geradkettige und verzweigte Alkylgruppen mit 1-7 C-Atomen, wie Methyl, Aethyl und Propyl, vorzugsweise Methyl. Der Ausdruck "nieder Alkoxy" bezieht sich auf Gruppen mit 1-7 C-Atomen, wie Methoxy und Aethoxy. Der Ausdruck "nieder Alkancarbonsäuren" bezieht sich auf Alkancarbonsäuren mit 1-7 C-Atomen, wie Ameisensäure und Essigsäure. Die Ausdrücke "Halogen" oder "Halo" beziehen sich auf alle Halogenatome, wie Fluor, Chlor, Brom und Jod.

Der Ausdruck "Aryl" bezeichnet eine einkernige aromatische Kohlenwasserstoffgruppe, wie Phenyl, die unsubstituiert oder in einer oder mehreren Stellungen durch nieder Alkylendioxy, Halogen, Nitro, nieder Alkyl oder nieder Alkoxy substituiert sein kann; und mehrkernige Arylgruppen, wie Naphthyl, Anthryl, Phenanthryl und Azulyl, die durch eine oder mehrere der oben erwähten Gruppen substituiert sein können. Die bevorzugten Arylgruppen sind substituierte und unsubstituierte einkernige Arylgruppen, insbesondere Phenyl. Der Ausdruck "Aryl-nieder-alkyl" umfasst Gruppen, in denen Aryl und nieder Alkyl wie oben definiert sind, insbesondere Benzyl. Der Ausdruck "Aryl-nieder-alkancarbonsäure" umfasst Säuren, worin Aryl und nieder-Alkancarbonsäure wie oben definiert sind, insbesondere Benzoesäure.

Der Ausdruck "hydrolysierbare Schutzgruppe" bezeichnet jede Schutzgruppe die unter Bildung der Gruppe, die sie schützt hydrolysier werden kann. Hydroxygruppen können durch Bildung hydrolysierbarer Ester, Acetale, Ketale oder Aether geschützt werden. Beispiele von Gruppen, die geeignet sind, durch Bildung von Estern organische Säuren zu schützen, sind nieder Alkylgruppen oder Halo-nieder-alkylgruppen. Eine geeignete Aetherschutzgruppe ist beispielsweise ein hydrolysierbarer nieder-Alkyläther, wie tert.Butyl und der Tetrahydropyranyläther. Andere Aethergruppen sind Arylmethyläther wie Benzyl, Benzhydryl oder Trityläther oder α-nieder--Alkoxy-nieder-alkyläther, beispielsweise Methoxymethyl-,

Methoxypropyl- oder Allyläther oder tri(substituierte)--Silyläther, in denen der Substituent nieder-Alkyl und/oder Aryl ist, wie Trimethylsilyläther oder Dimethyl-tert.butylsilyläther und Dimethylphenyläther.

In den hier wiedergegebenen Strukturformeln bedeutet eine keilförmige Bindung (▼) einen Substituenten, der ß-Orientierung hat (oberhalb der Molekülebene) eine unterbrochene Linie (≡) bedeutet einen Substituenten der in α-Stellung steht (unterhalb der Molekülebene) und eine Wellenlinie (∿∿) bedeutet einen Substituenten, der entweder α- oder ß-Stellung aufweist, oder Gemische dieser Isomeren.

In anderer Hinsicht betrifft die vorliegende Erfindung ein Verfahren zur Herstellung einer Verbindung der Formel I, worin Z >C=O ist, Diastereomere davon, in denen die ring--ständigen Substituenten die entgegengesetzte Stereokonfiguration von der in der Formel I angegebenen aufweisen und Gemische davon. Das erfindungsgemässe Verfahren ist dadurch gekennzeichnet, dass man eine Verbindung der Formel

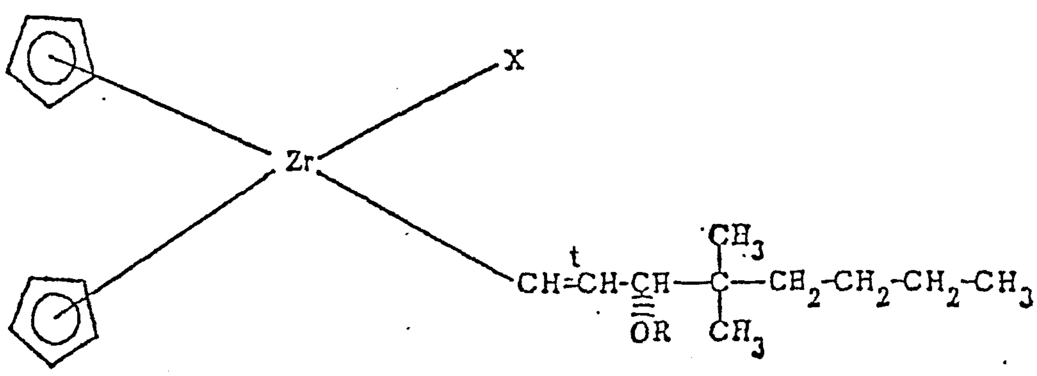

VI

worin X Halogen ist und R und t die obige Bedeutung haben,

mit einer Verbindung der Formel

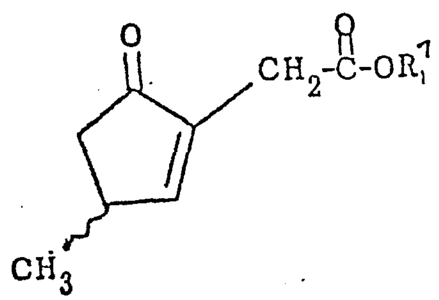

III

worin $R^1$ die obige Bedeutung hat, in einem wasserfreien inerten organischen Lösungsmittel, das als Katalysator ein Salz oder ein Komplex eines Uebergangsmetalls enthält, umsetzt und danach das Reaktionsprodukt mit einem Protonierungsmittel behandelt.

In dem obigen Verfahren wird eine Verbindung der Formel VI mit einer Verbindung der Formel III unter Bildung einer Verbindung der Formel VII-A oder VII-B

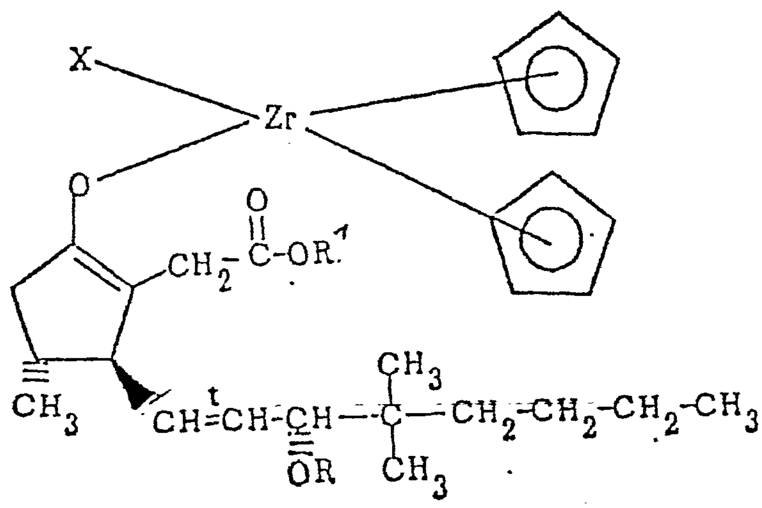

VII-A

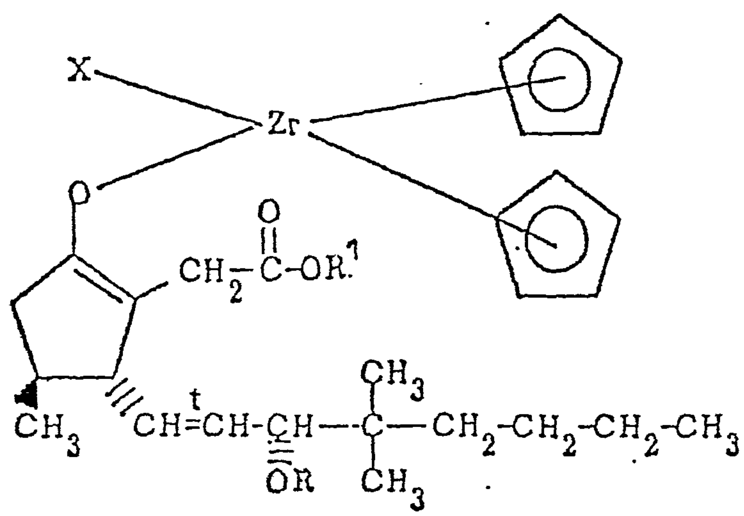

VII-B

oder Gemischen davon umgesetzt.

Wenn die Methylgruppe am Cyclopentenring der Verbindung der Formel III α-Stellung aufweist, erhält man bei dieser

Reaktion eine Verbindung der Formel VII-A. Andererseits erhält man ausgehend von Verbindungen in denen die Methylgruppe am Cyclopentenring der Verbindung der Formel III ß-Stellung aufweist, Verbindungen der Formel VII-B. Falls die Verbindung der Formel III in Form eines Gemisches der α- und ß-Methylverbindungen vorliegt, führt diese Reaktion zu einem Gemisch der Verbindung der Formel VII-A und VII-B. Die Umsetzung mit dem Zirconreagens der Formel VI führt somit die geschützte 3-Hydroxy-4,4-dimethyl-1-octenyl--Seitenkette in stereospezifischer Weise ein, die von der Stereokonfiguration der Methylgruppe am Cyclopentenylring der Verbindung der Formel III abhängt. Mittels dieser Reaktion werden somit die Methylgruppe und die Octenylseitenkette in den Cyclopentylring in stereospezifischer trans--Beziehung zueinander eingeführt.

Jedes konventionelle inerte organische Lösungsmittel kann bei dieser Reaktion verwendet werden, wobei aromatische Kohlenwasserstoffe, wie Benzol und Toluol, und ätherische Lösungsmittel, wie Tetrahydrofuran, besonders bevorzugt sind. Bei der Durchführung der Reaktion können Temperaturen von -20 bis 50°C verwendet werden, wobei Temperaturen von -10 bis 25°C besonders bevorzugt sind.

Die Umsetzung einer Verbindung der Formel VI mit einer Verbindung der Formel III wird in Gegenwart eines Salzes oder Komplexes eines Uebergangsmetallkatalysators durchgeführt. Unter diesen Katalysatoren sind Salze oder Komplexe von Nickel, Kobalt, Eisen, Mangan und Palladium. Bei der Durchführung der Reaktion können konventionelle Salze oder Komplexe der Uebergangsmetalle verwendet werden. Im allgemeinen ist es vorzuziehen, die Reaktion unter Verwendung eines Komplexes oder Salzes von Nickel(I) als Katalysator auszuführen, der in situ in der Reaktion erzeugt wird durch Verwendung eines Nickel(II)salzes oder Komplexes und eines Reduktionsmittels. Konventionelle Nickel(II)komplexe oder Salze und Reduktionsmittel können verwendet werden, um den

Nickel(I)katalysator zu erzeugen. Unter den bevorzugten Nickel(II)komplexen oder Salzen zur Bildung des Nickel(I)-katalysators ist Nickel(II)acetylacetonat. Unter den bevorzugten Reduktionsmitteln ist Diisobutylaluminiumhydrid (DIBAL).

Die Verbindungen der Formeln VII-A und VII-B oder Gemische davon werden in die entsprechenden Verbindungen der Formel I umgewandelt, in denen Z >C=O ist, durch Behandlung mit einem Protonierungsmittel. Jedes konventionelle Protonierungsmittel kann bei dieser Reaktion verwendet werden. Unter den konventionellen Protonierungsmitteln sind wasserfreie oder wässrige organische oder anorganische Säuren, wie Chlorwasserstoffsäure, Bromwasserstoffsäure, Schwefelsäure und Essigsäure, zu erwähnen. Diese Protonierung wird vorzugsweise in wasserfreiem Medium vorgenommen. Die Reaktion wird in einem konventionellen inerten organischen Lösungsmittel durchgeführt. Unter den bevorzugten inerten organischen Lösungsmitteln sind Aether, wie Tetrahydrofuran, Diäthyläther, Dioxan; oder Toluol und Benzol. Das bevorzugte Protonierungsmittel ist Chlorwasserstoffsäure in wasserfreier Form, d.h. Chlorwasserstoff. Bei der Durchführung der Reaktion sind Temperatur und Druck nicht kritisch, die Reaktion kann bei Zimmertemperatur ausgeführt werden.

Protonierung der Verbindung der Formel VII-A und/oder VII-B führt zur Verbindung der Formel I, in der Z >C=O ist und/oder den entsprechenden Diastereomeren, wobei die Estergruppe am Cyclopentylrest auf der gleichen Seite wie der Methylsubstituent steht.

Die Protonierung führt infolgedessen die Estergruppe in den Cyclopentenring in stereospezifischer Weise ein. Durch die Protonierung mit einer Halogenwasserstoffsäure erhält man Bis(cyclopentadienyl)zirkoniumdihalogenid als Nebenprodukt. Dieses Zirkoniumhalogenid kann aus dem Reaktionsgemisch zurückgewonnen werden und verwendet werden, um das

Zirkonreagens der Formel X herzustellen.

Ein anderer Aspekt der vorliegenden Erfindung betrifft ein Verfahren zur Herstellung einer Verbindung der Formel

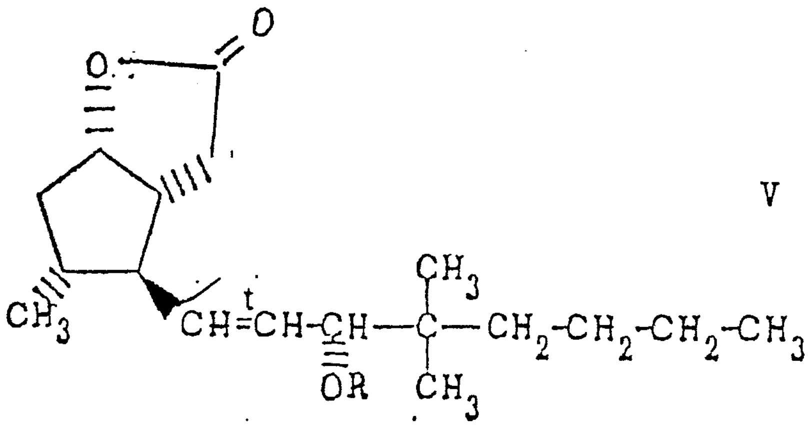

V

worin R und t die obige Bedeutung haben, Diastereomere davon, in denen die ring-ständigen Substituenten die entgegengesetzte Stereokonfiguration von der oben angegebenen haben und Gemische davon. Dieses Verfahren ist dadurch gekennzeichnet, dass man eine Verbindung der Formel I, worin Z >C=O ist, Diastereomere davon, in denen die ring-ständigen Substituenten die entgegengesetzte Stereokonfiguration von der oben angegebenen haben und Gemische davon, mit einem Borhydrid- oder Aluminiumhydrid-Reduktionsmittel in Gegenwart eines Deaktivierungsmittels für dieses Aluminiumhydrid-Reduktionsmittel behandelt.

Reduktion einer Verbindung der Formel V mit Aluminium oder Borhydrid-Reduktionsmitteln reduziert die Ketogruppe stereospezifisch zur Hydroxygruppe, sodass die Hydroxygruppe in α-Stellung am Cyclopentanring steht. Wenn andererseits die Ketogruppe in einem Diastereomeren der Verbindung der Formel V reduziert wird, wird die Hydroxygruppe in ß-Stellung am Cyclopentanring gebildet. Die Reduktion wird mittels eines Aluminium- oder Borhydrid-Reduktionsmittels ausgeführt. Unter den bevorzugten Reduktionsmitteln sind Dialkylaluminiumhydrid- und Alkalimetallborhydrid-Reduktionsmittel. Jedes konventionelle Dialkylaluminiumhydrid-Reduktionsmittel

kann verwendet werden, wobei Diisobutylaluminiumhydrid besonders bevorzugt ist. Bei der Durchführung der Reaktion unter Verwendung eines Aluminiumhydrid-Reduktionsmittels wird im allgemeinen eine organische Verbindung verwendet, die konventionell Aluminiumhydrid-Reduktionsmittel deaktiviert. Jede für die Deaktivierung von Aluminiumhydrid-Reduktionsmitteln konventionelle organische Verbindung kann für den erfindungsgemässen Zweck verwendet werden. Unter den bevorzugten Deaktivierungsmitteln sind di- oder tri(alkyl)-substituierte Phenole, wie 2,6-Di-tert.butyl-4-methylphenol und 2,3,6-Trimethylphenol. Von den Alkalimetallborhydriden sind Kalium-tris(sek.butyl)borhydrid und Natriumborhydrid bevorzugt. Bei der Durchführung der Reaktion werden im allgemeinen Temperaturen von -30 bis 10°C verwendet. Mit Alkalimetallaluminiumhydrid-Reduktionsmitteln, wie Lithiumaluminiumhydrid, wendet man vorzugsweise sehr niedrige Temperaturen an. Bei dieser Reaktion kann jedes konventionelle inerte organische Lösungsmittel, das bei -30 bis 10°C flüssig ist, verwendet werden. Unter den bevorzugten Lösungsmitteln sind aromatische Kohlenwasserstoffe, wie Toluol und Benzol.

Bei der Reduktion werden Verbindungen der Formel I, worin Z 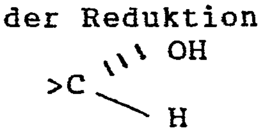 ist und deren Diastereomere, in denen die ring-ständigen Substituenten die entgegengesetzte Stereokonfiguration haben, als transitorische Zwischenprodukte erhalten, die unter den Reduktionsbedingungen in Lactone der Formel V und deren entsprechende Diastereomere übergehen. Die Verbindungen der Formel I, in denen Z 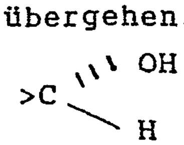 ist lactonisieren spezifisch zu Verbindungen der Formel V und das Diastereomere der Verbindung der Formel I lactonisiert stereospezifisch zum Diastereomeren der Verbindung der Formel V.

Wenn man eine Verbindung der Formel III verwendet, in

der die Methylgruppe in α-Stellung zur Ebene des Cyclopentanringes steht, verlaufen die verschiedenen Reaktionen zur Herstellung einer Verbindung der Formel V stereospezifisch. Die Verbindung der Formel V kann direkt in ein anti-sekretorisches Mittel der Formel

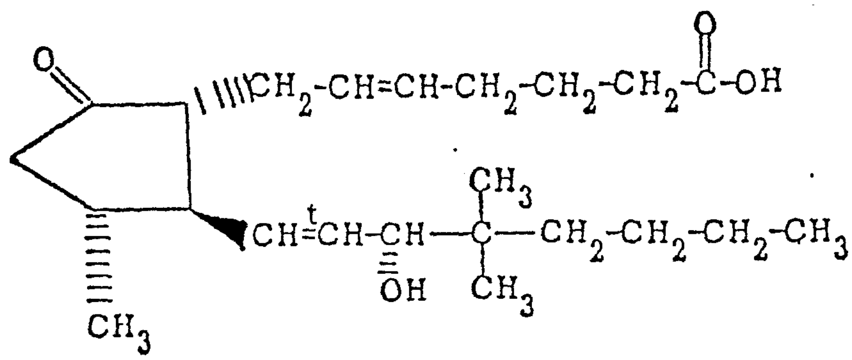

IV

gemäss der U.S. Patentschrift Nr. 4.227.019 umgewandelt werden.

Die vorliegende Synthese stellt damit eine Methode dar, zur asymmetrischen Gewinnung einer Verbindung der Formel IV ohne eine Racematspaltung durchführen müssen. Wenn andererseits die Verbindung der Formel III ein Gemisch der α- und ß-Isomeren ist, erhält man Gemische von Verbindungen der Formel I und deren Diastereomeren. Diese Gemische können in die individuellen Verbindungen durch konventionelle Mittel wie Chromatographie aufgetrennt werden.

Die folgenden Beispiele erläutern die Erfindung ohne sie zu beschränken. In den Beispielen wurde das Jones-Reagens durch Einwägen von 134 g Chromtrioxid in ein 500 ml-Volumetriegefäss und Zusatz von 200 ml Wasser hergestellt. Das Gemisch wurde unter langsamem Zusatz von 120 ml wässriger Schwefelsäure gerührt, die erhaltene Mischung auf Raumtemperatur abgekühlt und auf 500 ml mit Wasser aufgefüllt. Alle Temperaturen sind in Celsiusgraden angegeben.

## Beispiel 1

Ein 1-l-Dreihals-Rundkolben mit mechanischem Rührer, einem 250 ml-Tropftrichter für konstante Zugabe und Gaseinleitungsrohr wurde unter Inertgasatmosphäre flammen-getrocknet. Nach Abkühlen wurden 130 g (445 mMol) Dichlorbis-(eta$^5$-2,4-cyclopentadien-1-yl)zirkonium eingefüllt. Auf das feste Dichlorid wurden durch eine Kanüle 400 ml trockenes entgastes Toluol gegeben. In den Tropftrichter wurde durch eine Kanüle eine Lösung von Lithiumaluminiumhydrid--bis-tetrahydrofuran-Komplex in Toluol (103 ml, 111 mMol) gegeben. Die konstante Zugabe erforderte 1 Stunde und wurde im Dunkeln ausgeführt. Das erhaltene blassrosa-weisse Gemische wurde 2 Stunden gerührt, danach unter Inertgasatmosphäre durch eine grobe Fritte filtriert. Der Feststoff wurde mit dreimal 100 ml frisch destilliertem Tetrahydrofuran gewaschen und im Dunkeln bei 0,1 mmHg über Nacht (15 Stunden) bei 25° getrocknet und lieferte 97 g (85%) schneeweisses Chlor-bis(eta$^5$-2,4-cyclopentadien-1-yl)zirkoniumhydrid. Die Analyse auf dem Prozentsatz an überreduziertem Dihydrid, Bis(eta$^5$-2,4-cyclopentadien-1-yl)dihydrozirkonium, wurde durch Zusatz von 25 µl trockenem Aceton zu einem Gemisch von 0,5 ml Benzol-d-6 und 20 mg Produkt ausgeführt. Nach 90 Minuten Rühren wurde die klare farblose Lösung zur Analyse in ein NMR-Rohr gegeben. Die Analyse zeigte die Anwesenheit von 98.5% Monohydrid und 1.5% Dihydrid.

## Beispiel 2

4,4,-Dimethyl-1-octyn-3-on

In einen 3-l-Dreihals-Rundkolben mit mechanischem Rührer, Thermometer und Tropftrichter wurden 100,0 g (0,649 Mol) 4,4-Dimethyl-1-octin-3-ol und 1150 ml Aceton (Reagensqualität) gegeben. Der Tropftrichter wurde mit 241 ml frisch hergestelltem Jones-Reagens beschickt. Der Kolben wurde in einem Eismethanolbad auf -5° gekühlt. Das Jones-Reagens wurde langsam zugegeben, wobei die Temperatur unterhalb 10°

gehalten wurde. Die Zugabe erforderte etwa 20 Minuten. Das Reaktionsgemisch wurde weitere 10 Minuten gerührt, und dann in ein Gemisch von 2,2 l Petroläther und 2,2 l Eiswasser gegossen. Das Gemisch wurde 5 Minuten gerührt und dann in einen Scheidetrichter gegeben. Die organische Phase wurde abgetrennt und die wässrige Phase mit dreimal 700 ml Petroläther extrahiert. Die vereinigten organischen Phasen wurden mit 200 ml Wasser, 300 ml Kochsalzlösung gewaschen und über Natriumsulfat getrocknet. Das Lösungsmittel wurde unter vermindertem Druck in einem Rotationsverdampfer entfernt, wobei 98,5 g Rohprodukt erhalten wurden. Die gaschromatographische Analyse zeigte die Anwesenheit von 0,1% des als Ausgangsmaterials verwendeten Alkohols. Destillation bei 17 mmHg und 78° lieferte vier Fraktionen.

| Fraktion | g | % Alkohol | % Ausbeute |
|---|---|---|---|
| 1 | 4 | - | - |
| 2 | 78,5 | 0,08 | 79% |
| 3 | 8,4 | 0,29 | 8% |
| 4 | 1,9 | 0,70 | |

## Beispiel 3

(R)-4,4-Dimethyl-1-octin-3-ol

In einen flammen-getrockneten, mit Stickstoff gefüllten Dreihalb-Rundkolben von 5 l Inhalt mit Rückflusskühler, einem statischen Stickstoffeinleitungsrohr, Thermometer und magnetischem Rührer wurden 245 ml (0,783 Mol) Lithiumaluminiumhydrid-bis(diäthyläther)-Komplex als 3,2M-Lösung in Toluol gegeben. Eine Lösung von 191 g (1,56 Mol) 3,5-Dimethylphenol in 800 ml Diäthyläther (aus frisch geöffneter Flasche über basisches Aluminiumoxid unter Stickstoffatmosphäre geleitet) wurden in den Tropftrichter gegeben und im Verlauf von 2,5 Stunden langsam zu der Lithiumaluminiumhydridlösung gegeben. Das Reaktionsgemisch wurde bei Zimmertemperatur 1,5 Stunden gerührt, wobei 140 g (0,782 Mol) N-Methyl-d-ephedrin im Verlauf von 2,5 Stunden zugegeben

wurden. Das erhaltene Gemisch wurde über Nacht bei Raumtemperatur gerührt. Am nächsten Morgen wurde das Reaktionsgemisch auf -17 bis -22° gekühlt und mit einer Lösung von 66,6 g (0,438 Mol) 4,4-Dimethyl-1-octin-3-on in 900 ml Diäthyläther im Verlauf von 105 Minuten versetzt. Das Reaktionsgemisch wurde weitere 15 Minuten gerührt. Danach wurde die Reaktion durch vorsichtigen Zusatz von 30 ml Wasser abgebrochen. Nachdem die Gasentwicklung beendet war, wurde das Kühlbad entfernt und es wurden 1,2 l 3N Salzsäure zugesetzt. Nach 10 Minuten Rühren wurde die organische Phase abgetrennt und mit 800 ml 3N Salzsäure gewaschen. Die vereinigten sauren Phasen wurden mit dreimal 400 ml Aether extrahiert und zur Rückgewinnung des Amins bei Seite gestellt. Die vereinigten ätherischen Schichten wurden mit zweimal 500 ml 10% Natriumhydroxid und sechsmal 500 ml 15% Natriumhydroxid gewaschen. Gaschromatographische Analyse zeigte nur Spuren von verbliebenem Phenol. Die organische Phase wurde mit 500 ml Wasser und 500 ml Kochsalzlösung gewaschen und über Natriumsulfat getrocknet. Der Aether wurde unter vermindertem Druck in einem Rotationsverdampfer entfernt. Durch Destillation bei 40 mmHg wurde das Toluol entfernt. Der Druck wurde dann auf 9 mmHg abgesenkt und es wurden 58,3 g (86%) des Produktes bei 78° gesammelt. Gaschromatographische Analyse zeigte eine Reinheit von 96% an. Die gaschromatographische Analyse des (-)-α-Methoxy-α-trifluormethylphenylacetatderivats des Produkts zeigte eine Diastereomerenreinheit von 98% (R)-4,4-Dimethyl-1-octin-3-ol.

## Beispiel 4

rac-(R)-2-[(1-Aethinyl-2,2-dimethylhexyl)oxy]-tetrahydro-2H-pyran

In einen trockenen, mit Stickstoff gefüllten 1000-ml-Zweihals-Rundkolben mit magnetischem Rührer, statischem Stickstoffeinleitungsrohr und Rückflusskühler wurden 539 g (6,39 Mol) frisch destilliertes Dihydropyran, 363 g (2,36 Mol) (R)-4,4-Dimethyl-octin-1,3-ol und 1,0 g (5,26 mMol)

p-Toluolsulfonsäure-monohydrat gegeben. Das Reaktionsgemisch wurde in einem Oelbad von 105-115° 2 Stunden erhitzt. Nach Kühlen auf Raumtemperatur wurden 46 g (0,33 Mol) Kaliumcarbonat zugegeben. Der Kühler wurde durch einen Destillationsaufsatz ersetzt und das überschüssige Dihydropyran bei 50 mmHg entfernt. Das Produkt wurde bei 3 mmHg und 90-105° destilliert und wurde dabei in einer Ausbeute von 518 g (92%) als farblose Flüssigkeit, $[\alpha]_D^{25}$ = 105,4 (c = 1, Methanol) erhalten.

## Beispiel 5

In einen mit Argon gespülten Kolben wurden 15,9 g (66,8 mMol) rac-(R*)-2-[(1-Aethinyl-2,2-dimethylhexyl)oxy] -tetrahydro-2H-pyran und 55 ml frisch destilliertes Tetrahydrofuran gegeben. Zu dieser Lösung wurden 16,3 g (62,4 mMol) Chlorbis(eta$^5$-2,4-cyclopentadien-1-yl)zirkoniumhydrid bei 20° in kleinen Portionen gegeben, wobei man den Feststoff zwischen den Zusätzen in Lösung gehen liess. Die Zugabe erforderte 4 Stunden. Die Lösung wurde weitere 2 Stunden bei 20° gerührt, bevor das Tetrahydrofuran unter vermindertem Druck entfernt wurde. Insgesamt wurden 54 ml Tetrahydrofuran zurückgewonnen. Das (E,R)-Chlorbis(eta$^5$-2,4-cyclopentadien-1-yl)-[(3-tetrahydro -2H-pyran-2-yloxy)-4,4-dimethyl-1-octenyl]zirkonium (das Vinylzirkoniumreagens) wurde für die nächste Umsetzung in 10 ml Tetrahydrofuran gelöst.

## Beispiel 6

474 g (3,04 Mol) (S)-(-)-Citronellal in 2,37 l Methanol wurden bei -65° während 4,5 Stunden ozonisiert. Das Lösungsmittel wurde unter vermindertem Druck bei 45° entfernt, der Rückstand in 3,79 l 90%iger Ameisensäure gelöst und mit 200 ml 30%igem Wasserstoffperoxid versetzt. Das Gemisch wurde auf 55° zur Einleitung der Reaktion erwärmt, und mit 500 ml Ameisensäure und anschliessend tropfenweise mit 1,7 l 30%igem Wasserstoffperoxid versetzt. Die Geschwindigkeit der

Zugabe wurde so reguliert, dass ein schwaches Rückflussieden aufrechterhalten wurde. Das Gemisch wurde über Nacht bei Raumtemperatur gerührt, mit 1 l gesättigter Kochsalzlösung verdünnt und mit dreimal 1 l Aethylacetat extrahiert. Der Extrakt wurde über Magnesiumsulfat getrocknet und unter vermindertem Druck eingedampft, wobei 468,7 g rohe (S)-3--Methyladipinsäure erhalten wurden. Diese wurde durch Auflösen in 1,5 l Acetonitril und anschliessendem Zusatz von 268 g Morpholin gereinigt. Das Gemisch wurde bei Raumtemperatur über Nacht gerührt und die Kristalle durch Filtration gesammelt. Die Kristalle wurden in 200 ml 25%iger Chlorwasserstoffsäure gelöst, das Gemisch mit dreimal 1 l Aethylacetat extrahiert, der Extrakt über Magnesiumsulfat getrocknet und eingedampft. Man erhielt (S)-(-)-3-Methyladipinsäure vom Smp. 80-83°; $[\alpha]_D^{25}$ = -5,95° (Aethylacetat, c = 0,97).

## Beispiel 7

Eine Lösung von 320 g (2,0 Mol) (S)-(-)-3-Methyladipinsäure in 1,5 l Methylenchlorid wurde auf -20° gekühlt und mit 1,5 l kaltem (-20°) Isobutylen und anschliessend mit 20 ml konzentrierter Schwefelsäure versetzt. Das Gemisch wurde unter einem Druck von 200 psi Stickstoff während 20 Stunden gehalten und dann in 1 l gesättigtes Natriumbicarbonat gegossen. Die organische Phase wurde gesammelt, über Magnesiumsulfat getrocknet und eingedampft und lieferte 443 g (S)-(-)-3-Methyl-hexandisäure-bis(1,1-dimethyläthyl)-ester, $[\alpha]_D^{25}$ = -3,34°.

## Beispiel 8

Ein Gemisch von 84,0 g (2,15 Mol) Natriumamid und 1966 ml Toluol wurde unter Rühren und unter Inertgasatmosphäre tropfenweise mit einer Lösung von 4,11 g (1,51 Mol) (S)-3-Methylhexandisäure-bis(1,1-dimethyläthyl)ester in 500 ml wasserfreiem Toluol versetzt. Das Gemisch wurde 4 Stunden bei Rückflusstemperatur und dann über Nacht bei

Raumtemperatur gerührt, mit 200 ml Toluol verdünnt, mit 242 ml Essigsäure angesäuert und schliesslich mit 500 ml Wasser verdünnt. Die organische Phase wurde gesammelt, mit 500 ml gesättigter Kochsalzlösung gewaschen, getrocknet und eingedampft. Man erhielt 234 g 2-[(1,1-Dimethyläthoxy)carbonyl]-(S)-4-methylcyclopentanon, das mit ca. 10% 2-[(1,1-Dimethyläthoxy)carbonyl]-(S)-3-methylcyclopentanon verunreinigt war. Sdp. 76-82°/0,25 mm.

## Beispiel 9

Zu 269 g (1,35 Mol) 2-Carboäthoxy-(S)-4-methylcyclopentanon wurden 145,3 g (1,425 Mol) Aethylglyoxylat gegeben. Das Gemisch wurde über Nacht bei Raumtemperatur gerührt und lieferte rohen α-Hydroxy-(S)-4-methyl-1-[(1,1-dimethyläthoxy)carbonyl] -2-oxocyclopentanessigsäure-äthylester als viskoses Oel. Ein reines Präparat wurde durch Chromatographie an Silicagel mit 10% Aethylacetat in Hexan gewonnen.

## Beispiel 10

Eine Lösung von 406,5 g (1,347 Mol) α-Hydroxy-(S)-4-methyl-1-[(1,1-dimethyläthoxy)carbonyl] -2-oxocyclopentanessigsäure-äthylester in 627 g Trifluoressigsäure wurde 2 Stunden bei Raumtemperatur gerührt und unter vermindertem Druck bei 40° eingeengt. Der Rückstand wurde mit 1 l Aethylacetat extrahiert, mit 1,75 l gesättigter Natriumbicarbonatlösung gewaschen und die wässrige Phase mit zweimal 60 ml Aethylacetat rückextrahiert. Die vereinigten Extrakte wurden über Magnesiumsulfat getrocknet und eingedampft und lieferte 261 g α-Hydroxy-(S)-4-methyl-2-oxocyclopentan-essigsäure -äthylester, Sdp. 95-97°/0,05 mmHg.

## Beispiel 11

Eine Lösung des in Beispiel 10 erhaltenen α-Hydroxy-(S)-4-methyl-2-oxocyclopentan-essigsäure -äthylesters in

3,9 l Cyclohexan wurde mit 12,30 g p-Toluolsulfonsäure-monohydrat versetzt. Das Gemisch wurde unter Rühren 3 Stunden zum Rückfluss erhitzt, wobei das Wasser azeotrop entfernt wurde. Danach wurde auf Raumtempratur gekühlt und mit 400 ml gesättigter Natriumbicarbonatlösung gewaschen. Die organische Phase wurde abgetrennt, mit 200 ml gesättigter Kochsalzlösung gewaschen und getrocknet und eingedampft. Man erhielt 185 g rohen (R)-4-Methyl-2-oxo-cyclopentylidenessigsäure-äthylester.

Beispiel 12

Eine Lösung von 184 g (1,01 Mol) (R)-4-Methyl-2-oxo-cyclopentylidenessigsäure-äthylester in 1,84 l Toluol wurde mit 17,3 g 4-Dimethylaminopyridin versetzt. Das Gemisch wurde über Nacht zum Rückfluss erhitzt, auf Raumtemperatur abgekühlt und mit 400 ml 1N Salzsäure, 200 ml 10% Natriumbicarbonatlösung und 300 ml gesättigter Kochsalzlösung gewaschen. Die organische Phase wurde über Magnesiumsulfat getrocknet und eingedampft. Man erhielt 192 g Rohprodukt, das durch Chromatographie an 1,5 kg Silicagel mit 10% Aethylacetat in Hexan gereinigt wurde. Man erhielt 92,8 g reines (R)-3-Methyl-5-oxo-1-cyclopenten-1-essigsäure-äthylester, Sdp. 80-90°/0,03 mm; $[\alpha]_D^{25}$ = +91,79° (Chloroform, c = 0,99).

Beispiel 13

Ein Gemisch von 0,98 g (3,8 mMol) frisch sublimiertes und getrocknetes Nickel(II)acetylacetonat und 8 ml frisch destillierter Tetrahydrofuran wurde auf -6° gekühlt und unter Rühren mit 3,8 ml (3,8 mMol) Diisobutylaluminiumhydrid (DIBAH) im Verlauf von 1 Minute versetzt. Danach wurden 10,3 g (54,3 mMol, 95% gaschromatographisch rein) sauerstoffreies (R)-3-Methyl-5-oxo-1-cyclopenten-1-essigsäure-äthylester (das Enon) in einen auf dem Reaktionsgefäss angebrachten Tropftrichter gegeben. 5 Minuten nach Zusatz

von DIBAH wurde das Enon im Verlauf von 5 Minuten zu der Nickel(I)-Lösung gegeben. Danach wurden im Verlauf von 5 Minuten das in Beispiel 5 hergestellte Vinylzirkoniumreagens in Tetrahydrofuran gegeben und mit 5 ml Tetrahydrofuran nachgespült. Danach wurde das Reaktionsgemisch 17 Stunden bei einer konstanten Badtemperatur von 15° gerührt. Gaschromatographische Analyse zeigte danach einen 97%igen Umsatz an. Das Reaktionsgemisch wurde auf -15° abgekühlt und mit 20,5 ml (78 mMol, 1,25 Aequivalente bezogen auf Zirkonium) von wasserfreiem 3,8N Salzsäure in Tetrahydrofuran gegeben. (Diese Lösung wurde durch Einleiten von wasserfreiem HCl in kaltes Tetrahydrofuran und Titration mit Natriumhydroxidlösung frisch hergestellt). Nach 5 Minuten wurden 75 ml Tetrahydrofuran zugegeben, um die Filtration zu erleichtern. Das Gemisch wurde unter einer Inertgasatmosphäre durch eine mittlere Fritte, die mit 10 g wasserfreiem Natriumbicarbonat bedeckt war, unter Inertgasatmosphäre filtriert. Das Reaktionsgefäss wurde mit zweimal 75 ml Tetrahydrofuran gespült und die Lösung durch die gleiche Filterschicht gegeben. Die Tetrahydrofuranlösung wurde dann an einem Rotationsverdampfer konzentriert, der Rückstand mit 250 ml Hexan versetzt, kräftig umgerührt und der weisse Feststoff absitzen gelassen. Der Feststoff wurde dann mit weiteren 250 ml Hexan aufgenommen und filtriert. Nach Trocknen erhielt man 19 g (theoretisch 18,2 g) weisses Bis(cyclopentadienyl)zirkoniumdichlorid. Die vereinigten Hexanwaschwässer wurden filtriert, wobei 50 mg fein suspendierte Feststoffe entfernt wurden und unter vermindertem Druck zu einem grünen viskosen Oel konzentriert. Der Hauptteil des durch Protonolyse von überschüssigem Vinylzirkoniumreagens gebildeten Olefins wurde bei 80-100° und 0,1 mmHg entfernt. Das Rohprodukt, [1S-[1α,2β(1E,3R*),3α]-2-[3-[(tetrahydro-2H-pyran -2-yl)oxy]-4.4-dimethyl-1-octenyl]-3-methyl -5-oxocyclopentanessigsäure-äthylester wurde gaschromatographisch als 86% rein ermittelt. Die Ausbeute betrug 23,8 g (etwa 48 mMol [89%] bezogen auf gaschromatograpisch reines Produkt).

Dieses Rohprodukt wurde im nächsten Schritt verwendet.

Beispiel 14

Zu 47,0 g (211 mMol) 2,6-Di-tert.butyl-4-methylphenol (BHT) wurden 267 ml Toluol aus einem frisch geöffneten Gefäss gegeben. Das Gemisch wurde gerührt und die erhaltene Lösung durch Einleiten von Argon sauerstoffrei gemacht. Die Lösung wurde auf -5° gekühlt und im Verlauf von 30 Minuten bei einer Temperatur zwischen 0° und 5° mit 175 ml Diisobutylaluminiumhydrid (0,6 mMol/ml in Toluol, 105 mMol) versetzt. Die erhaltene klare farblose Lösung wurde 2 Stunden bei -2° gerührt. 23,75 g des gemäss Beispiel 13 hergestellten rohen [1S-[1α,2β(1E,3R*),3α]-2-[3-[(tetrahydro-2H--pyran -2-yl)oxy]-4.4-dimethyl-1-octenyl]-3-methyl -5-oxocyclopentanessigsäure-äthylesters wurden in 25 ml Toluol gelöst, wie vorstehend beschrieben, sauerstoffrei gemacht und nach Kühlen auf -15° im Verlauf von 30 Minuten zugesetzt. Das Reaktionsgemisch wurde weitere 2 Stunden gerührt und danach mit 13 ml gesättigter Natriumsulfatlösung versetzt. Das Kühlbad wurde entfernt und das Reaktionsgemisch 30 Minuten gerührt. Das Gemisch wurde abfiltriert und mit zweimal 50 ml Toluol gewaschen. Die gelbe Lösung wurde über Natriumsulfat getrocknet und das Toluol bei 40-50° und 10 mmHg entfernt. Der Rückstand wurde schliesslich bei 5 mmHg auf 100-110° 20 Minuten lang erwärmt. Man erhielt 70 g rohes Lacton, das gemäss HPLC-Analyse ein Diastereomerengemisch im Verhältnis 9:1 von [3aR,6aS,4R,3'R,5R,E]-3,3a,4,5,6,6a-Hexahydro-4 -[4,4-dimethyl-3-[(tetrahydro-2H-pyran-2-yl)oxy]-1--octenyl] -5-methyl-2H-cyclopenta[b]furan-2-on und [3aS,-6aR,4S,3'R,5S,E]-3,3a,4,5,6,6a-Hexahydro-4 -[4,4-dimethyl-3--[(tetrahydro-2H-pyran-2-yl)oxy]-1-octenyl] -5-methyl-2H-cyclopenta[b]furan-2-on darstellte. Das BHT wurde durch Chromatographie einer 40%igen Lösung in Hexan, das 1% Aethylacetat enthielt, an 125 g Silicagel entfernt. Die Säule wurde mit 600 ml dieses Lösungsmittels und 1 l Hexan, das 50% Aethylacetat enthielt, eluiert. Das BHT war in der

1%-Aethylacetat-Fraktion, und das Lacton in der 50% Aethylacetat-enthaltenden Fraktion. Die Lacton-Fraktion wurde bei 100° und 15 mmHg konzentriert und lieferte 16,4 g des oben genannten Diastereomerengemisches in einer gaschromatographischen Reinheit von 99%, Ausbeute 79% bezogen auf Cyclopentenon-Ausgangsmaterial.

## Beispiel 15

Ein Gemisch von 161 g (624,35 mMol) Chlorobis(eta$^5$--2,4-cyclopentadien-1-yl)zirkoniumhydrid, 163 g (684,53 mMol) rac-(R)-2-[(1-Aethinyl-2,2-dimethylhexyl)loxy]tetrahydro -2H-pyran und 208 ml frisch destilliertem Tetrahydrofuran wurde 15 Minuten reagieren gelassen, dann von 30°C auf 23°C abgekühlt und während 2 Stunden jede 15 Minuten abgekühlt, bis die Reaktion nicht mehr exothermisch verlief. Nach 4 Stunden entstand eine rötlich-orange Lösung enthaltend (E,R)-Chlorobis(eta$^5$-2,4-cyclopentadien-1-yl) -[(3--tetrahydro-2H-pyran-2-yloxy)-4,4-dimethyl -1-octenyl]zirkonium.

19 g (73,96 mMol) frisch sublimiertes und getrocknetes Nickel(II)acetylacetonat und 208 ml frisch destilliertes Tetrahydrofuran wurde unter Argonatmosphäre gerührt und auf -15° gekühlt. Im Verlauf von 5 Minuten wurden 65 ml (71,5 mMol) Diisobutylaluminiumhydrid (DIBAL) in Toluol zugegeben. Nachdem Zusatz war die Temperatur auf 15° gestiegen, worauf das Reaktionsgemisch erneut auf -10° rasch abgekühlt wurde. Nach 5 Minuten wurden im Verlauf von 5 Minuten 87,2 g (478,6 mMol) (R)-3-Methyl-5-oxo-1-cyclopentenyl-1-essigsäure-äthylester (99% rein) zugesetzt. Die in Beispiel 5 hergestellte Lösung von (E,R)-Chlorobis(eta$^5$-2,4-cyclopentadien-1-yl) -[(3-tetrahydro-2H-pyran-2-yloxy)-4,4-dimethyl -1-octenyl]-zirkonium wurde 5 Minuten nach Zusatz des Cyclopentenderivats im Verlauf von 30 Minuten so zugegeben, dass die Reaktionstemperatur unterhalb 0° blieb. Danach wurde die Reaktionsmischung 23 Stunden bei -6° gehalten.

Das Reaktionsgemisch, dass das Zirkoniumenolat, [3R-[3ß,(1E,3R*),4α]]-Chlorobis(eta$^5$-2,4-cyclopentadien -1-yl)[2-(2-äthoxy-2-oxoäthyl)-3-[tetrahydro-2H-pyran -2-yl)oxy]-4,4-dimethyl-1-octenyl]-4-methyl-1-cyclopenten -1-olatozirkonium(-1) enthielt, wurde auf -15° gekühlt und danach mit 180 ml (780,44 mMol) 4,45N HCl in Tetrahydrofuran (1,25 Aequivalente pro Zirkonium) versetzt. Diese Lösung wurde frisch hergestellt durch Einleiten von wasserfreiem Chlorwasserstoff in kaltes gerührtes Tetrahydrofuran und Titrieren mit Natriumhydroxidlösung. Nach 5 Minuten wurde das viskose Gemisch durch eine Filterschicht von 35 g Natriumbicarbonat unter Argonatmosphäre filtriert. Das Reaktionsgefäss wurde mit fünfmal 100 ml Tetrahydrofuran gewaschen und diese Lösung ebenfalls durch das Bicarbonatfilter gegeben. Das Filtrat wurde vom Lösungsmittel befreit und der Rückstand mit zweimal 250 ml Hexan gewaschen. Bei jeder Wäsche wurde das Gemisch kräftig gerührt bevor sich die Kristallmasse absetzte. Die Hexanphase, die eine feine Suspension dunkler Feststoffe enthielt, wurde vom weissen Feststoff abdekantiert. Die erste Hexanwaschlösung wurde abfiltriert und lieferte 24,3 g eines grüngrau-schwarzen Feststoffes von Bis(cyclopentadienyl)zirkoniumdichlorid, die zweite Filtration lieferte 20,15 g dieses Zirkoniumchlorids. In der filtrierten Hexanlösung bildete sich ein Niederschlag. Die Lösung musste zweimal filtriert werden, wobei 3,3 g und 9,2 g Zirkoniumdichlorid erhalten wurden bevor die Hexanlösung klar blieb. Insgesamt wurden 189 g (theoretisch 182,5 g) Zirkoniumdichlorid zurückgewonnen. Die Hexanschichten wurden gesammelt und vom Lösungsmittel befreit und lieferten [1S-[1α,2ß(1E,3R*),3α]]-2-[3-[tetrahydro-2H--pyran -2-yl)oxy]-4,4-dimethyl-1-octenyl]-3-methyl -5-oxo--cyclopentanessigsäure-äthylester als grünes viskoses Oel. Das durch Protonierung von überschüssigem Vinylzirkoniumreagens gebildete Olefin wurde bei 80-110° und 5 mmHg abdestilliert. Das Produkt war 93% gaschromatographisch rein. Die

Ausbeute betrug 215 g (98%).

Beispiel 16

Ein Gemisch von 426 g (1,93 Mol) 2,6-Di-tert.butyl-4--methylphenol (BHT) und 2,3 l Toluol wurde gerührt und durch Einleiten von Argon während 20 Minuten sauerstoffrei gemacht. Nach Kühlen auf -5° wurden 898 ml Diisobutylaluminiumhydrid (DIBAL) in Toluol (1,1 mMol/ml) im Verlauf von 30 Minuten so zugegeben, dass die Temperatur zwischen -5° und 0° blieb. Die klare farblose Lösung, die Bis[2,6-di--tert.butyl-4-methylphenoxy]aluminiumhydrid enthielt, wurde 2 Stunden unter Argon bei -2° gerührt. Zu dieser Lösung wurden die in Beispiel 15 erhaltenen 215 g [1S-[1α,2β(1E,-3R*),3α]]-2-[3-[tetrahydro-2H-pyran -2-yl)oxy]-4,4-dimethyl-1-octenyl]-3-methyl -5-oxo-cyclopentanessigsäure-äthylester in 200 ml Toluol, die zuvor wie oben beschrieben, sauerstoffrei gemacht worden war im Verlauf von 30 Minuten zu der auf -15° gekühlten Lösung gegeben. Das Reaktionsgemisch wurde danach noch 2 Stunden gerührt und die Lösung bei -15° zu 50 ml gesättigter wässriger Natriumsulfatlösung gegeben. Die Dünnschichtchromatographie zeigte eine unvollständige Cyclisierung an. Die Lösung wurde aus dem Kühlbad herausgenommen und weitere 50 ml gesättigte Natriumsulfatlösung wurden zugesetzt, wobei die Temperatur auf 25° stieg. Danach zeigte die Dünnschichchromatographie vollständige Cyclisation an mit zwei grösseren und zwei kleineren Flecken (THP Diastereomere). Nach 30 Minuten Rühren bei 25° wurde das Gemisch filtriert, mit zweimal 200 ml Toluol gewaschen, über Natriumsulfat getrocknet und filtriert. Das Toluol wurde bei 10 mmHg und 40° Badtemperatur entfernt, um das restliche Toluol durch 20 Minuten langes Erhitzen auf 100-110° bei 5 mmHg. Man erhielt 613 g rohes [3aR,6aS,4R,-3'R,5R,5]-3,3a,4,5,6,6a-Hexahydro]-4 -[4,4-dimethyl-3--[(tetrahydro-2H-pyran-2-yl)oxy] -1-octenyl]-5-methyl-2H--cyclopenta[b]furan-2-on. Die Analyse dieses Rohproduktes zeigte ein Diastereomerenverhältnis von 92:8. Das BHT wurde

durch Chromatographie einer 40 Gew.-%igen Lösung des Rohproduktes an Silicagel (Lösungsmittel Hexan mit 19 Vol.-% Aethylacetat) entfernt. Die Säule wurde einer Gradient-Elution mit Hexan/Aethylacetat in der nachstehend angeführten Reihenfolge unterworfen.

| Konzentration Aethylacetat | Anzahl Fraktionen | Fraktions-volumen | Fraktions-zahl |
|---|---|---|---|
| 1% | 10 | 1 l | 1-10 |
| 2% | 7 | 1 l | 11-17 |
| 10% | 7 | 1 l | 18-24 |
| 20% | 6 | 1 l | 25-30 |
| 30% | 6 | 1 l | 31-36 |
| 40% | 5 | 1 l | 36-40 |

Die das gewünschte enthaltenden Fraktionen waren die folgenden.

| Fraktionen | Gewicht | |
|---|---|---|
| 20-26 | 101,58 g | überwiegendes Diastereomer |
| 27-31 | 37,66 g | überwiegendes Diastereomer mit einer Spur des im geringeren Ausmass vorhandenen Diastereomers |
| 34-40 | 5,14 g | im geringeren Ausmass vorhandenes Diastereomer |
| | 144,38 g | Gesamtausbeute aller Isomeren 381,71 mMol |

Das BHT erschien in den Fraktionen 1, 2 und 3. Die gesammelten Fraktionen wurden vom Lösungsmittel befreit, Reste von Lösungsmittel wurden unter Hochvakuum bei 100° und 5 mmHg entfernt. Die Ausbeute an [3aR,6aS,4R,3'R,5R,E]-3,3a,4,5,6,6a-Hexahydro-4 -[4,4-dimethyl-3-[(tetrahydro-2H-pyran-2-yl)oxy] -1-octenyl]-5-methyl-2H-cyclopenta[b]furan-2-on betrug 80% bezogen auf Cyclopentenon-Ausgangsmaterial.

## Patentansprüche

1. Verbindungen der Formel

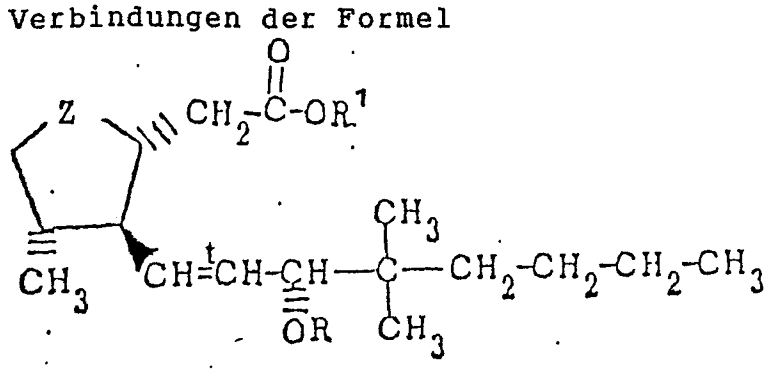

I.

worin Z >C=O oder 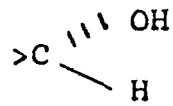 ; $R^1$ zusammen mit dem Sauerstoffatom, an das es gebunden ist, eine hydrolysierbare Estergruppe, R zusammen mit dem Sauerstoffatom, an das es gebunden ist, eine hydrolysierbare Aethergruppe darstellt und t trans-Konfiguration bezeichnet, Diastereomere davon, in denen die ring-ständigen Substituenten die entgegengesetzte Stereokonfiguration zu der oben angegebenen haben, und Gemische davon.

2. Verbindungen gemäss Anspruch 1, in denen Z >CO ist.

3. Verbindungen gemäss Anspruch 1, in denen Z 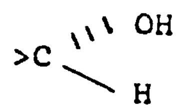 ist.

4. Verbindungen gemäss Anspruch 1, in denen R Tetrahydropyran-2-yl und $R^1$ Aethyl ist.

0153689

5. Verbindungen der Formel

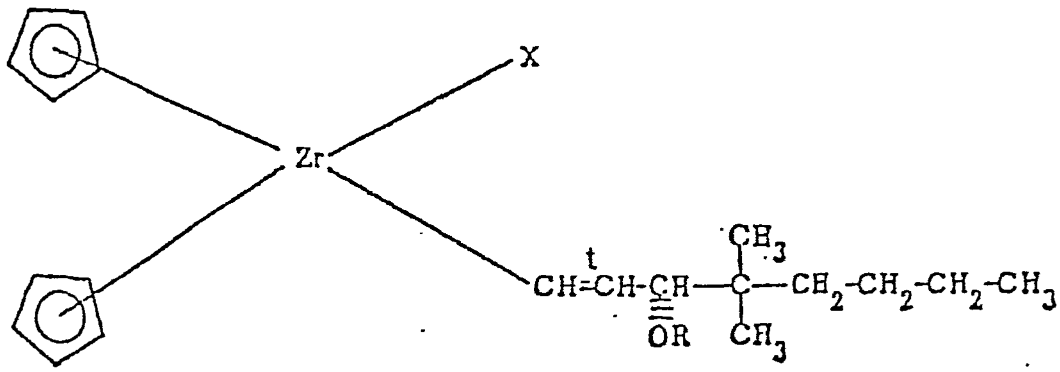

VI

worin X Halogen und R wie in Anspruch 1 ist.

6. Verbindungen gemäss Anspruch 5, in denen R Tetrahydropyran-2-yl ist.

7. Die Verbindung gemäss Anspruch 6, in der X Chlor ist.

8. Verfahren zur Herstellung einer Verbindung der Formel I, worin Z >C=O ist, Diastereomeren davon, in denen die ring-ständigen Substituenten die entgegengesetzte Stereokonfiguration von der in der Formel I angegebenen aufweisen und Gemischen davon, dadurch gekennzeichnet, dass man eine Verbindung der Formel

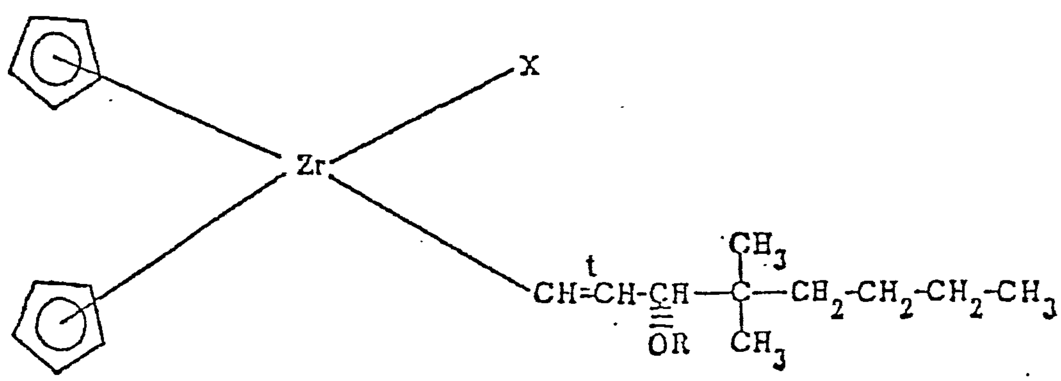

VI

worin X Halogen ist und R und t die in Anspruch 1 angegebene Bedeutung haben,

mit einer Verbindung der Formel

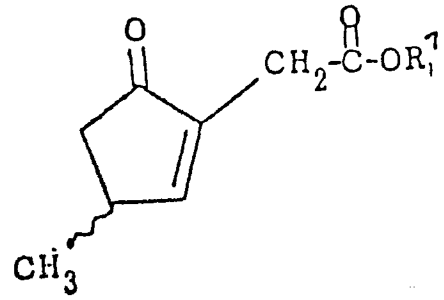

III

worin $R^1$ die in Anspruch 1 angegebene Bedeutung hat, in einem wasserfreien inerten organischen Lösungsmittel, das als Katalysator ein Salz oder ein Komplex eines Uebergangsmetalls enthält, umsetzt und danach das Reaktionsprodukt mit einem Protonierungsmittel behandelt.

9. Verfahren gemäss Anspruch 8, wobei der Katalysator ein Ni(I)-Komplex oder -Salz ist.

10. Verfahren gemäss Anspruch 8, wobei die Umsetzung einer Verbindung der Formel III mit einer Verbindung der Formel IV bei -20° bis 50°C durchgeführt wird.

11. Verfahren gemäss Anspruch 8, wobei das Protonierungsmittel Wasser ist.

12. Verfahren gemäss Anspruch 8, wobei das Protonierungsmittel eine organische oder anorganische Säure ist.

13. Verfahren zur Herstellung einer Verbindung der Formel

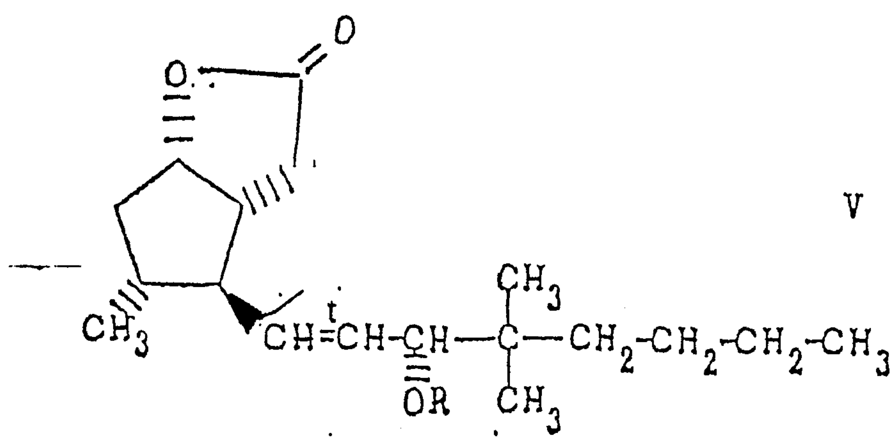

V

worin R und t die obige Bedeutung haben, Diastereomeren davon, in denen die ring-ständigen Substituenten die entgegengesetzte Stereokonfiguration von der oben angegebenen haben und Gemischen davon, dadurch gekennzeichnet, dass man eine Verbindung der Formel I, worin Z >C=O ist, Diastereomere davon, in denen die ring-ständigen Substituenten die entgegengesetzte Stereokonfiguration von der oben angegebenen haben und Gemische davon, mit einem Borhydrid- oder Aluminiumhydrid-Reduktionsmittel in Gegenwart eines Deaktivierungsmittels für das Aluminiumhydrid--Reduktionsmittel behandelt.

14. Verfahren gemäss Anspruch 13, wobei die Verbindungen I und V die angegebene Stereokonfiguration haben.

15. Verfahren gemäss Anspruch 13, wobei das Reduktionsmittel Diisobutylaluminiumhydrid und das Deaktivierungsmittel ein Di- oder Tri-(nieder-alkyl)-phenol ist.

***

0153689
DV 4303/13

## Patentansprüche für Oesterreich

1. Verfahren zur Herstellung einer Verbindung der Formel

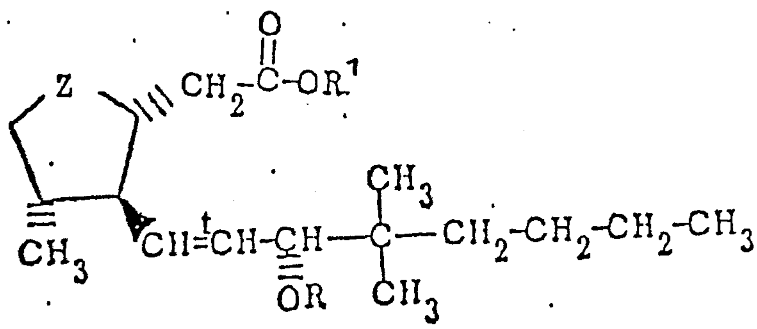

I.

worin Z >C=O; $R^1$ zusammen mit dem Sauerstoffatom, an das es gebunden ist, eine hydrolysierbare Estergruppe, R zusammen mit dem Sauerstoffatom, an das es gebunden ist, eine hydrolysierbare Aethergruppe darstellt und t trans-Konfiguration bezeichnet,

Diastereomeren davon, in denen die ring-ständigen Substituenten die entgegengesetzte Stereokonfiguration zu der oben angegebenen haben, und Gemischen davon, dadurch gekennzeichnet, dass man eine Verbindung der Formel

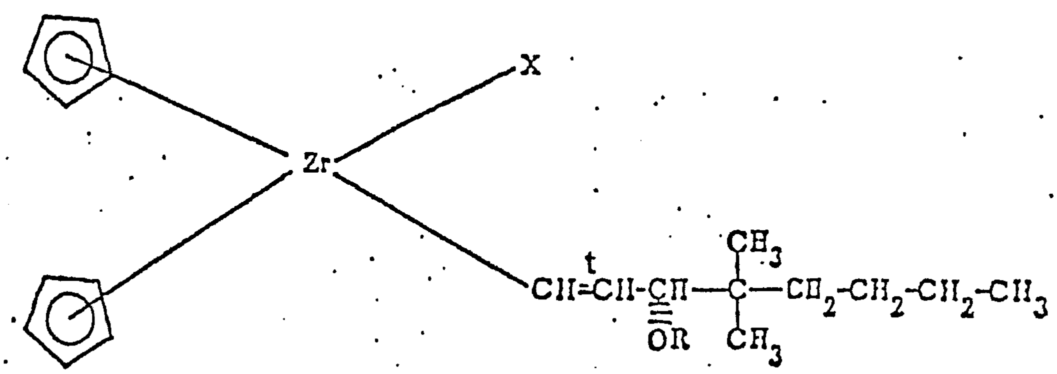

VI

worin X Halogen ist und R und t die oben angegebene Bedeutung haben,

mit einer Verbindung der Formel

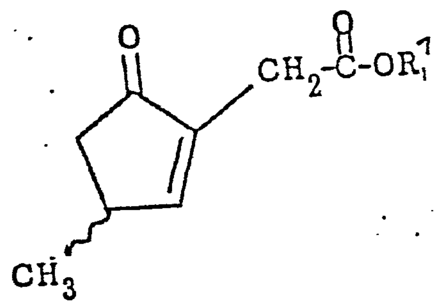

III

worin $R^1$ die oben angegebene Bedeutung hat, in einem wasserfreien inerten organischen Lösungsmittel, das als Katalysator ein Salz oder ein Komplex eines Uebergangsmetalls enthält, umsetzt und danach das Reaktionsprodukt mit einem Protonierungsmittel behandelt.

2. Verfahren gemäss Anspruch 1, wobei der Katalysator ein Ni(I)-Komplex oder -Salz ist.

3. Verfahren gemäss Anspruch 1, wobei die Umsetzung einer Verbindung der Formel III mit einer Verbindung der Formel IV bei -20° bis 50°C durchgeführt wird.

4. Verfahren gemäss Anspruch 1, wobei das Protonierungsmittel Wasser ist.

5. Verfahren gemäss Anspruch 1, wobei das Protonierungsmittel eine organische oder anorganische Säure ist.

6. Verfahren gemäss Anspruch 1, wobei R Tetrahydropyran-2-yl und $R^1$ Aethyl ist.

0153689

DV 4303/13

7. Verfahren zur Herstellung einer Verbindung der Formel

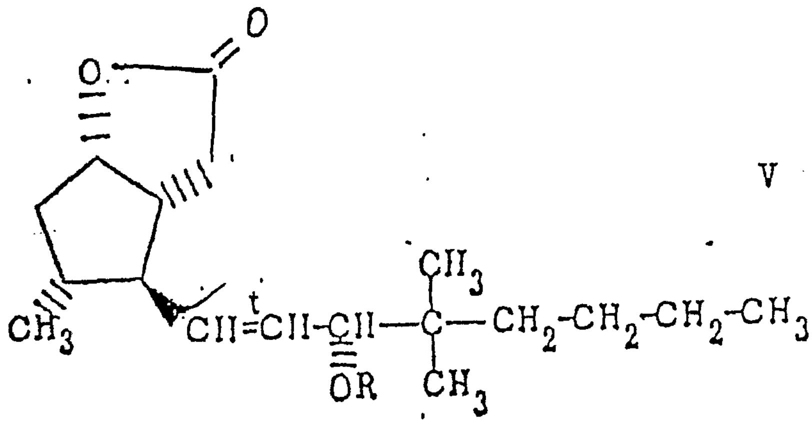

V

worin R und t die in Anspruch 1 angegebene Bedeutung haben,

Diastereomeren davon, in denen die ring-ständigen Substituenten die entgegengesetzte Stereokonfiguration von der oben angegebenen haben und Gemischen davon, dadurch gekennzeichnet, dass man eine Verbindung der Formel I, worin Z >C=O ist, Diastereomere davon, in denen die ring-ständigen Substituenten die entgegengesetzte Stereokonfiguration von der oben angegebenen haben und Gemische davon, mit einem Borhydrid- oder Aluminiumhydrid-Reduktionsmittel in Gegenwart eines Deaktivierungsmittels für das Aluminiumhydrid--Reduktionsmittel behandelt.

8. Verfahren gemäss Anspruch 7, wobei die Verbindungen I und V die angegebene Stereokonfiguration haben.

9. Verfahren gemäss Anspruch 7, wobei das Reduktionsmittel Diisobutylaluminiumhydrid und das Deaktivierungsmittel ein Di- oder Tri-(nieder-alkyl)-phenol ist.

***